# EUROPEAN PATENT SPECIFICATION

(11) **EP 1 455 781 B1**
(45) Date of publication and mention of the grant of the patent: **27.08.2008**
(21) Application number: 02783305.2
(22) Date of filing: 06.12.2002
(51) Int. Cl.: A61K 31/4196, A61P 35/00

(54) **USE OF ANASTROZOLE FOR THE TREATMENT OF POST-MENOPAUSAL WOMEN HAVING EARLY BREAST CANCER**
VERWENDUNG VON ANASTROZOL ZUR BEHANDLUNG VON POSTMENOPAUSALEN FRAUEN MIT FRÜHZEITIGEM BRUSTKREBS
UTILISATION DE L'ANASTROZOLE POUR LE TRAITEMENT DU CANCER DU SEIN A UN STADE PRECOCE CHEZ LES FEMMES MENOPAUSEES

(30) Priority: 10.12.2001 GB 0129457
(43) Date of publication of application: 15.09.2004
(73) Proprietor: AstraZeneca AB, 151 85 Södertälje (SE)
(72) Inventor: PLOURDE, Paul, Wilmington, DE 19850 (US); ROGERS, Anthony, Wilmington, DE 19850 (US); VOSE, Brent, Macclesfield, Cheshire SK10 4TG (GB); DUKES, Michael, Macclesfield, Cheshire SK10 4TG (GB)
(74) Representative: French, Philip Joseph
(86) International application number: PCT/GB2002/005554
(87) International publication number: WO 2003/053438

(56) References cited:
- WO-A-02/20000
- "PHARMACOKINETICS OF ANASTROZOLE AND TAMOXIFEN ALONE, AND IN COMBINATION, DURING ADJUVANT ENDOCRINE THERAPY FOR EARLY BREAST CANCER IN POSTMENOPAUSAL WOMEN: A SUB-PROTOCOL OF THE 'ARIMIDEX AND TAMOXIFEN ALONE OR IN COMBINATION' (ATAC) TRIAL" BRITISH JOURNAL OF CANCER, LONDON, GB, vol. 85, no. 3, 3 August 2001 (2001-08-03), pages 317-324, XP009005779 ISSN: 0007-0920
- BAUM M: "Use of aromatase inhibitors in the adjuvant treatment of breast cancer." ENDOCRINE-RELATED CANCER. ENGLAND JUN 1999, vol. 6, no. 2, June 1999 (1999-06), pages 231-234, XP002236966 ISSN: 1351-0088
- DOWSETT M ET AL: "A randomised, double blind parallel-group trial to evaluate the effect of 'Arimidex' (Anastrozole) on the pharmacokinetics of tamoxifen in postmenopausal breast cancer patients." BREAST CANCER RESEARCH AND TREATMENT, vol. 46, no. 1, October 1997 (1997-10), page 30 XP009008829 20th Annual San Antonio Breast Cancer Symposium;San Antonio, Texas, USA; December 3-6, 1997 ISSN: 0167-6806
- DOWSETT M ET AL: "The effect of anastrozole on the pharmacokinetics of tamoxifen in post - menopausal women with early breast cancer." BRITISH JOURNAL OF CANCER, (1999 JAN) 79 (2) 311-5. , XP009008911
- KUERER H M ET AL: "Biologic basis and evolving role of aromatase inhibitors in the management of invasive carcinoma of the breast." JOURNAL OF SURGICAL ONCOLOGY. UNITED STATES JUN 2001, vol. 77, no. 2, June 2001 (2001-06), pages 139-147, XP009008821 ISSN: 0022-4790
- NABHOLTZ J M ET AL: "Anastrozole is superior to tamoxifen as first-line therapy for advanced breast cancer in postmenopausal women: Results of a North American multicenter randomized trial." JOURNAL OF CLINICAL ONCOLOGY, vol. 18, no. 22, 15 November 2000 (2000-11-15), pages 3758-3767, XP002236968 ISSN: 0732-183X
- BONNETERRE JACQUES ET AL: "Anastrozole is superior to tamoxifen as first-line therapy in hormone receptor positive advanced breast carcinoma: Results of two randomized trials designed for combined analysis." CANCER, vol. 92, no. 9, 1 November 2001 (2001-11-01), pages 2247-2258, XP002236969 ISSN: 0008-543X
- SAINSBURY, R. (1) ET AL: "Anastrozole is superior to tamoxifen in the treatment of post - menopausal women with early breast cancer: First results of the ATAC (' Arimidex ', tamoxifen, alone or in combination) trial." INTERNATIONAL JOURNAL OF CANCER SUPPLEMENT, (2002) NO. 13, PP. 137. PRINT MEETING INFO.: 18TH UICC INTERNATIONAL CANCER CONGRESS OSLO, NORWAY JUNE 30-JULY 05, 2002 , XP002236970
- DUFFY, S. (1) ET AL: "The ATAC (' Arimidex ', tamoxifen, alone or in combination) early breast cancer trial in postmenopausal patients: Endometrial sub-protocol results." INTERNATIONAL JOURNAL OF CANCER SUPPLEMENT, (2002) NO. 13, PP. 137-138. PRINT. MEETING INFO.: 18TH UICC INTERNATIONAL CANCER CONGRESS OSLO, NORWAY JUNE 30-JULY 05, 2002 , XP002236971
- THE ATAC (ARIMIDEX TAMOXIFEN ALONE OR IN COMBINATION) TRIALISTS' GROUP: "Anastrozole alone or in combination with tamoxifen versus tamoxifen alone for adjuvant treatment of postmenopausal women with early breast cancer: first results of the ATAC randomised trial" LANCET, XX, XX, vol. 359, no. 9324, 22 June 2002 (2002-06-22), pages 2131-2139, XP004366986 ISSN: 0140-6736
- EBCTCG: "Tamoxifen for early breast cancer: an overview of the randomized trials" LANCET, vol. 351, 1998, pages 1415-1467,
- THE ATAC TRIALISTS GROUP: "Results of the ATAC trial after completion of 5 years adjuvant treatment for breast cancer" LANCET, vol. 365, 2005, pages 60-62

## Description

In post-menopausal women, the anti-oestrogen tamoxifen citrate (NOLVADEX™) has become accepted as standard first-line treatment of advanced breast cancer. Furthermore, over 34,000 women have taken part in clinical trials of adjuvant tamoxifen following primary surgery. The anti-cancer effect of tamoxifen citrate (hereinafter "tamoxifen") is related to its ability to compete with oestrogen for binding sites in target tissue such as the breast. In addition, it has a variety of other complicated mechanisms which may mediate its effect, including the induction of transforming growth factor beta from stromal fibroblasts, the reduction in circulating levels of insulin-like growth factor 1, the inhibition of angiogenesis and the induction of apoptosis. Nevertheless, the oestrogen receptor (ER) status of the tumour still remains a powerful predictor of response.

In advanced disease, approximately 70% of patients who are ER and progesterone receptor (PR) positive will respond to tamoxifen, compared with <10% of patients who are ER and PR negative (Muss, 1992 Breast Cancer Research and Treatment 21: 51-26). In the adjuvant setting, the ER status of the primary tumour is less discriminatory and the latest overview of the Early Breast Cancer Trialists' Co-operative Group (EBCTCG) [Lancet 351: 1451-1467] has suggested that only pre-menopausal ER negative patients are unlikely to benefit from adjuvant tamoxifen. It is likely that other endocrine approaches to the adjuvant treatment of early breast cancer, which involve reducing the level of circulating oestradiol (ovarian suppression and aromatase inhibition), are dependant on an intact oestrogen receptor mechanism at the sites of metastatic disease.

Aromatase inhibitors are a class of compounds that act systematically to inhibit oestrogen synthesis in tissues. These compounds prevent oestrogen biosynthesis by inhibiting the enzyme aromatase, which catalyses the conversion of adrenal androgens (androstenedione and testosterone) to oestrogens (oestrogen and oestradiol). There has therefore been interest in developing these compounds as potential therapies for hormone responsive breast cancer in post-menopausal women.

Aminoglutethimide was the first aromatase inhibitor to be approved for treatment of breast cancer and has proven efficacy in post-menopausal women with advanced breast cancer (Stuart-Harris at al 1984, Acta Oncology 27: 721-728). Wider use of the drug has been limited by its lack of specificity, resulting in a requirement for concomitant administration of corticosteroids, and the occurrence of troublesome side effects (Wells et al 1978, Annals Surgery 187: 475-487). Consequently, research has been focused on the development of aromatase inhibitors with greater specificity and a better tolerability profile.

ARIMIDEX™ (anastrozole) is a non-steroidal aromatase inhibitor which is highly selective, well tolerated and is effective in treating advanced breast cancer (Buzdar et al 1995, The Breast 4(3): 256-257 Abs 104; Jonat et al 1995, European Journal of Cancer 32A(3): 404-412; Plourde et al 1995, Journal of Steroid Biochemistry 53:175-179). (Further information on the clinical experience with Arimidex can be found in the prescribing information sheet for Arimidex). Anastrozole is described in U.S. patent RE 366717, which is incorporated by reference herein.

The use of adjuvant systemic therapies following local treatment of breast cancer (surgery with or without radiotherapy) has resulted in an increase in disease-free survival and overall survival (EBCTCG, 1992, Lancet 339: 1-15, 71-85). Tamoxifen is currently the agent of choice as adjuvant endocrine therapy for breast cancer <+>. Patients treated with adjuvant tamoxifen for two years or more can expect an annual reduction in recurrence of 39% and an annual reduction in mortality of 24%, in the absence of concomitant chemotherapy (EBCTCG, 1992). In pre-menopausal women with oestrogen receptor positive tumours, the benefit is as great as for post-menopausal women, but there seems little benefit for ER negative pre-menopausal women. In contrast, both ER positive and ER negative post-menopausal women will benefit, although there is a suggestion that the greater the ER level in the primary tumour the greater the likelihood of benefit.

Tamoxifen therapy also provides other beneficial effects, which relate to the partial agonistic action of the drug. These include cholesterol-lowering effects (Love et al 1994, Journal of the National Cancer Institute 86: 1534-1539), cardio-protective effects (McDonald et al 1995, British Medical Journal 311: 977-980) and protection against bone loss (Love et al 1992, New England Journal of Medicine 326: 852-856). However, adverse events are also associated with tamoxifen which can be classified as being either consequences of the anti-oestrogenic action of the drug, e.g. hot flushes, vaginal bleeding or discharge or dryness, or more general effects, e.g. gastro-intestinal intolerance, tumour flare, light headedness, skin rash.

An increased incidence of endometrial cancer has also been reported in association with tamoxifen treatment. The incidence and pattern of this increase suggest that the underlying mechanism may be related to the oestrogenic properties of tamoxifen. Uterine including early breast cancer (see EBC TCG, 1998, Lancet 351, 1415 - 1467) fibroids and other endometrial changes including hyperplasia and polyps have also been reported in patients receiving tamoxifen.

Notwithstanding the good results obtained with tamoxifen as an adjuvant therapy in patients with early breast cancer, there is a good rationale to evaluate alternative endocrine modalities in this patient population alone although it could be predicted that combinations therapies could be more effective. Benefits could include a longer duration of effectiveness and possible freedom from the side effects associated with the partial agonist properties of tamoxifen.

One possible combination is a combination of anastrozole and tamoxifen in the light of the proven efficacy of anastrozole in advanced breast cancer, its favourable tolerability and a mechanism of action distinct from tamoxifen.

An early trial, using the aromatase inhibitor aminoglutethimide alone, demonstrated improvements in relapse-free survival and overall survival, when compared to placebo (Coombes et al, 1987, Cancer Research 47, 2494-2497), but the supportive management required, and the restricted patient acceptability prevented further development. In contrast, anastrozole, in phase III trials for the second line treatment of advanced breast cancer, not only demonstrated similar efficacy to megestrol acetate, but also a favourable tolerability profile, especially with respect to weight gain.

NOLVADEX™ and ARIMIDEX™ are trademarks and are the property of the AstraZeneca group of companies.

Surprisingly we have found that anastrozole is efficacious and well tolerated in the adjuvant treatment of breast cancer, but even more surprisingly we have found that anastrozole is significantly more effective than tamoxifen for disease-free survival in early breast cancer. Thus described herein is the use of anastrozole to reduce the rate of recurrence of cancer in a post-menopausal woman having early breast cancer comprising administering an effective amount of anastrozole, or its pharmaceutically acceptable salt, to said woman.

According to a further feature of the first aspect of the invention there is provided the use of anastrozole, or a pharmaceutically acceptable salt thereof, in the preparation of a medicament for the reduction of the rate of recurrence of cancer in a post-menopausal woman having early breast cancer.

In women who are receiving adjuvant treatment for breast cancer, the cancer can re-emerge in the same breast as the original cancer or may occur in the other breast, referred to as contralateral breast cancer. Surprisingly we have also found that anastrozole was significantly more effective than tamoxifen in terms of incidence of contralateral breast cancer, thus described herein is the use of anastrozole to reduce the rate of a new contralateral primary tumour in a post-menopausal woman having early breast cancer comprising administering an effective amount of anastrozole, or its pharmaceutically acceptable salt, to a said woman.

According to a further feature of the second aspect of the invention there is provided the use of anastrozole, or a pharmaceutically acceptable salt thereof, in the preparation of a medicament for the reduction of the rate of a new contralateral primary tumour in a post-menopausal woman having early breast cancer.

Tamoxifen has been approved for use for adjuvant therapy for early breast cancer and thus it could be expected that anastrozole would be synergistic with tamoxifen, depleting the oestrogen receptor of its natural ligand, whilst allowing tamoxifen to exert its beneficial effect via alternative biological mechanisms. However, surprisingly, we have found that rather than being more effective than tamoxifen alone, a combination of anastrozole and tamoxifen was only equivalent to tamoxifen and significantly worse than anastrozole alone for adjuvant treatment of early breast cancer. Thus, surprisingly tamoxifen reduces the efficacy of anastrozole for the adjuvant treatment of early breast cancer. Thus, described hered is use of anastrozole to reduce the rate of recurrence of cancer in a post-menopausal woman having early breast cancer comprising administering an effective amount of anastrozole or its pharmaceutically acceptable salt to said woman wherein the anastrozole or its pharmaceutically acceptable salt is administered in the substantial absence of tamoxifen.

According to a further feature of the third aspect of the invention there is provided the use of anastrozole or a pharmaceutically acceptable salt thereof, substantially in the absence of tamoxifen in the preparation of a medicament for the reduction of the rate of recurrence of cancer in a post-menopausal woman having early breast cancer.

The phrase 'substantially in the absence of tamoxifen' means that the anastrozole is administered to the patient in the absence of tamoxifen. Patients receiving adjuvant therapy for early breast cancer are likely to have previously received tamoxifen treatment. Thus, this phrase relates to the anastrozole medication rather than the patient being treated.

Surprisingly we have also found that the combination of anastrozole and tamoxifen was only equivalent to tamoxifen and significantly worse than anastrozole alone in the prevention of contralateral breast cancer. Thus, discribed herein is the use of anastrozole to reduce the rate of a new contralateral primary tumour in a post-menopausal woman having early breast cancer comprising administering an effective amount of anastrozole or its pharmaceutically acceptable salt to said woman wherein the anastrozole or its pharmaceutically acceptable salt is administered in the substantial absence of tamoxifen.

According to a further feature of the fourth aspect of the invention there is provided the use of anastrozole or a pharmaceutically acceptable salt thereof substantially in the absence of tamoxifen in the preparation of a medicament for the reduction of the rate of a new contralateral primary tumour in a post-menopausal woman having early breast cancer.

In each aspect of the invention the anastrozole, or its pharmaceutically acceptable salt is preferably administered as an adjuvant to surgery, chemotherapy or radiation. Surgery includes lumpectomy, quandrantectomy or mastectomy.

In another aspect of the invention, the anastrozole or its pharmaceutically acceptable salt is administered to a woman having early breast cancer and the woman is oestrogen receptor positive or progesterone receptor positive, more preferably oestrogen receptor positive, most preferably oestrogen receptor positive and progesterone receptor positive.

Anastrozole can be administered at a dose between 0.1 and 10 mg/day, preferably, between 0.5 and 5mg/day, most preferably anastrozole is administered at 1mg/day.

As described above in one aspect of the invention the anastrozole, or its pharmaceutically acceptable salt, is administered in the substantial absence of tamoxifen. However, in a further feature of the third and fourth aspect of the invention the anastrozole is administered in the substantial absence of an anti-oestrogen agent such as arzoxifene, tamoxifen, fulvestrant, lasofoxifene, raloxifene, toremifene, trilostane or TSE 424, preferably in the absence of tamoxifen.

Treatment of post-menopausal women is preferred as described above, however, treatment of pre-menopausal women is also contemplated.

In one aspect of the invention, the administration is carried continuously, for a least one year, preferably for at least two years. Five years continuous treatment is most preferred, although greater than five years treatment is also contemplated.

In carrying out the present invention, anastrozole may be provided in various formulations such as parentally (e.g. aqueous or oily suspensions) or orally (e.g., tablets, powders, capsules, granules, aqueous or oily suspensions). Preferably, anastrozole is provided in orally available formulations preferably in formulations having from 1 to 10mg anastrozole, most preferably 1mg. In the study described below, 1mg tablets were used.

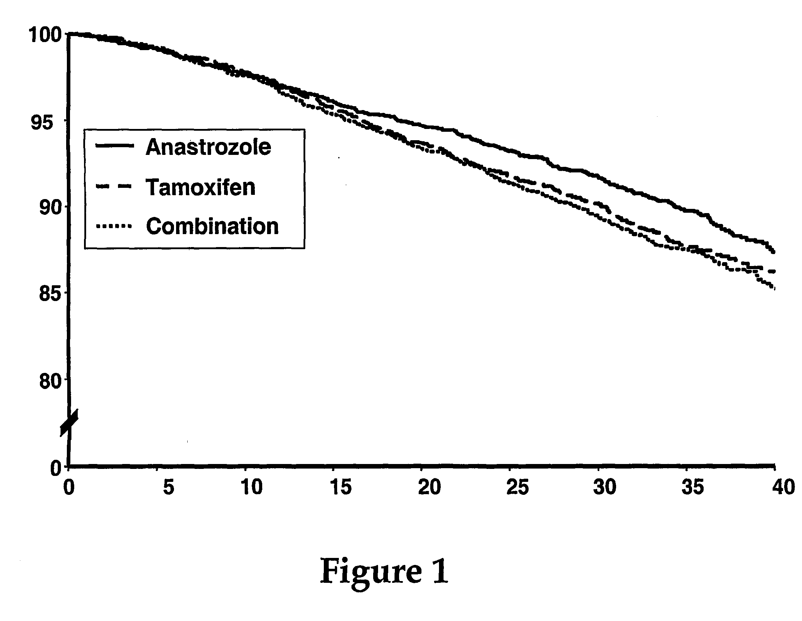

The invention will now be illustrated with reference to the following example and the following figures and tables. In this figure legends HR is an abbreviation of hazard ratio. Values wherein the hazard ratio is less that 1.00 mean the values are in favour of anastrozole or the combination of anastrozole and tamoxifen as appropriate. Values wherein the hazard ratio is greater than 1.00 means that the values are in favour of tamoxifen. The 'time to events' is the time between the commencement of receiving therapy within the trial and diagnosis of recurrence of breast cancer. Figures 1-3 depict Kaplan-Meier Curves which would be familiar to the skilled man, however, more information can be found in Modelling Survival Data in Medical Research by D.Collett (Published by Chapman & Hall, 1994).
Figure 1 - Kaplan-Meier Curves of disease-free survival in ITT population
The Figure shows 'Time to events (months)' on the X axis and 'Proportion of patients event free (%)' on the Y axis.

| | **HR** | **95.2% CI** | **p-value** |
|---|---|---|---|
| **anastrozole vs tamoxifen** | 0.83 | 0.71-0.96 | 0.0129 |
| **combination vs tamoxifen** | 1.02 | 0.88-1.18 | 0.7718 |

This figure shows that in the overall population, anastrozole is superior to tamoxifen with a significant reduction in the rate of disease recurrence, the combination is similar to that of tamoxifen for the overall population.

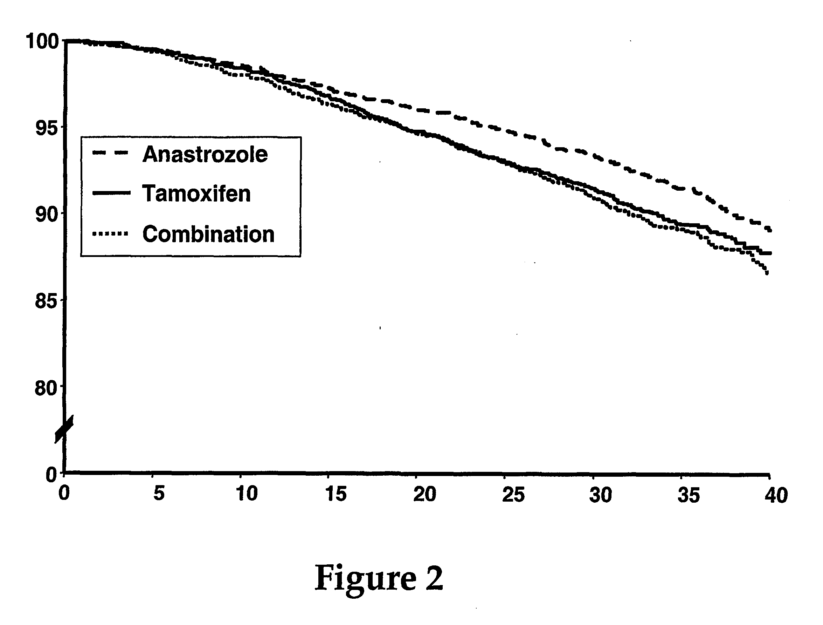

Figure 2 - Kaplan-Meier Curves of disease-free survival in receptor positive population
The Figure shows 'Time to events (months)' on the X axis and 'Proportion of patients event free (%)' on the Y axis.

| | **HR** | **95.2% CI** | **P-value** |
|---|---|---|---|
| **anastrozole vs tamoxifen** | 0.78 | 0.65-0.93 | 0.0054 |
| **combination vs tamoxifen** | 1.02 | 0.87-1.21 | 0.7786 |

This figures shows that in the overall population, anastrozole is superior to tamoxifen with a significant reduction in the rate of disease recurrence, the combination is similar to that of tamoxifen for the overall population.

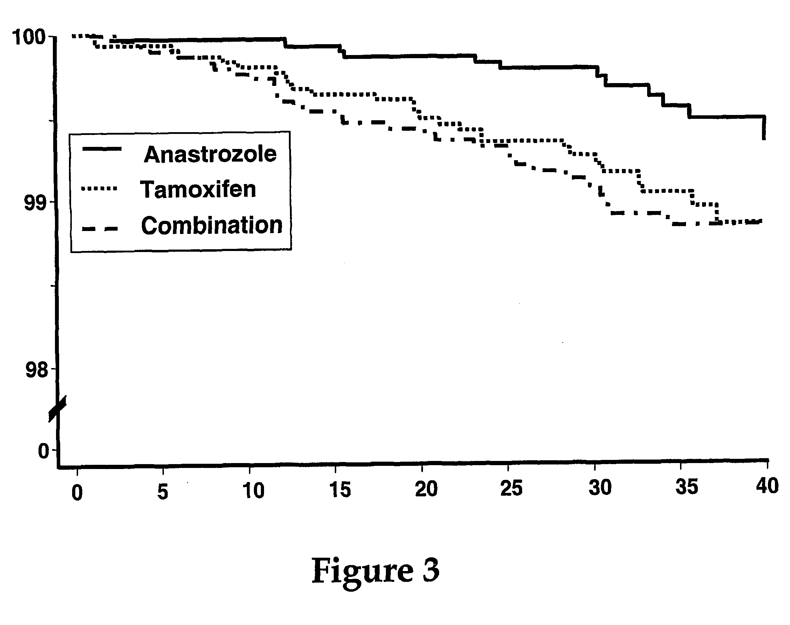

Figure 3 - Analysis of the incidence of new (contralateral) breast primaries;
The figure shows 'Time to first contralateral new primary (months)' on the X axis and 'Proportion without contralateral breast cancer (%)' on the Y axis.

| | **HR** | **95.2% CI** | **P-value** |
|---|---|---|---|
| **anastrozole vs tamoxifen** | 0.42 | 0.22-0.79 | 0.0068 |
| **combination vs tamoxifen** | 0.84 | 0.51-1.40 | 0.5132 |

This figues shows the superiority of anastrazole is also evident in terms of the incidence of new primary (contralateral) breast cancer, relative to tamoxifen, whereas the combination is similar to tamoxifen.

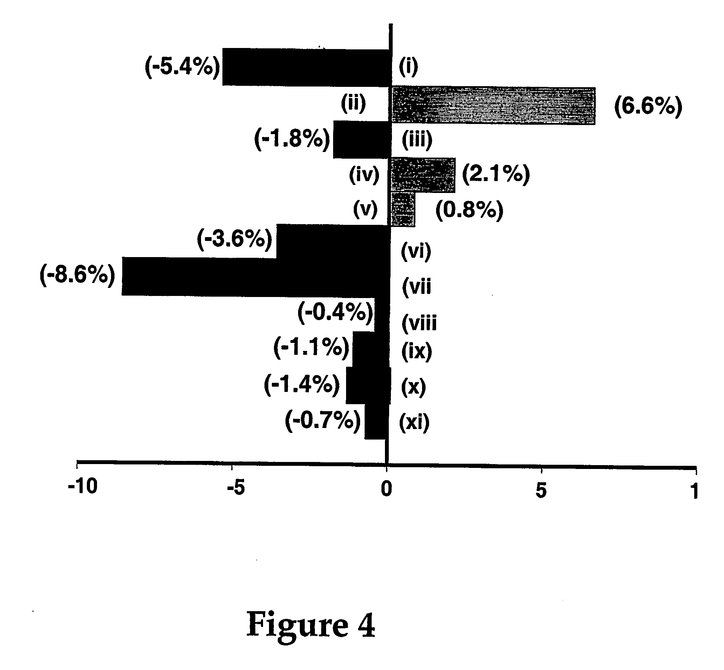

Figure 4 - Significant differences in pre-defined adverse events between anastrozole and
tamoxifen expressed as a percent. Adverse events less prevalent in anastrozole are shown on the left hand side of the figure and adverse events less prevalent with tamoxifen are shown on the right of the figure. The adverse events shown are as follows:
(i) hot flushes
(ii) Musculoskeletal Disorders
(iii) Weight gain (proportion with ≥10% gain in body weight from baseline in year 2)
(iv) Fractures of any bone
(v) Fractures of hip, spine , wrist (these factures are indicators of osteoporosis)
(vi) Vaginal bleeding
(vii) Vaginal discharge
(viii) Endometrial Cancer
(ix) Ischaemic Cardiovascular Accidents
(x) Venous Thromboembolic Events
(xi) Deep Vein Thrombosis
This figure shows that Anastrozole is well tolerated and no unexpected safety concerns emerged in the adjuvant programme.
Table 1 - Summary of the characteristics of the patients in the trial;
Table 2 - Summary of the disease characteristics of the patients in the trial; and
Table 3 - First events in ITT population.
   Abbreviations: CI- confidence interval;
   HR - hazard ratio;
   ITT - intention to treat;
   od - once daily; and
   OR - odds ratio.

A trial was designed and conducted to compare the efficacy and safety of tamoxifen and anastrozole alone and with anastrozole and tamoxifen in combination as adjuvant treatment for post-menopausal women with early breast cancer, who have completed their primary therapy. The trial was designed to compare tamoxifen (20mg once daily [od]) and anastrozole (1mg od) and to compare tamoxifen (20mg od) and the combination of anastrozole (1mg od) plus tamoxifen (20mg od) as adjuvant treatment in terms of:
a) time to recurrence of breast cancer (defined as the earliest of local or distant recurrence, new primary breast cancer, or death); and
b) safety and side effects.
Secondary objectives of this trial were to compare (i) tamoxifen and anastrozole and (ii) to compare tamoxifen and the combination of anastrozole plus tamoxifen as adjuvant treatment in terms of:
a) time to distant recurrence;
b) survival; and
c) incidence of new breast primaries.

Specifically, the trial was designed as a randomised, double-blind, multicentre trial to compare the efficacy and safety of tamoxifen alone, anastrozole alone and anastrozole in combination with tamoxifen as adjuvant treatment for breast cancer in post-menopausal women. Patients who meet the eligibility criteria were randomised on a 1:1:1 basis into one of three oral treatment schedules to receive one of the following:
a. active anastrozole 1mg once daily plus tamoxifen placebo once daily;
b. active tamoxifen 20mg once daily plus anastrozole placebo once daily;
c. active anastrozole 1mg once daily in combination with active tamoxifen 20mg once daily.

Patients were assessed at entry, 3 months, 6 months and thereafter at 6-month intervals. Patients were recruited from 381 centres and in 21 countries. The patients were recruited between July 1996 and March 2000. Nine thousand three hundred and sixty-six (9366) patients entered the trial and were given the drugs as follows: 1) Anastrozole (n=3125), 2) tamoxifen (n=2116) and 3) combination (n=3125).

In order to be eligible for entry into the trial the patients had to satisfy all the following:
a) patients with histologically proven operable invasive breast cancer;
b) patients who have completed all primary surgery and chemotherapy (if given), and are candidates to receive hormonal adjuvant therapy; and
c) women defined as post-menopausal according to one or more of the following:-
   (i) aged 60 or more
   (ii)aged 45-59 years and satisfying one or more of the following criteria:
      - amenorrhoea for at least 12 months and intact uterus;
      - amenorrhoea for less that 12 months and FSH within the post-menopausal range; including:
         - patients who have had a hysterectomy;
         - patients who have received HRT;
         - patients rendered amenorrhoetic by adjuvant chemotherapy (such patients must have FSH measured at least 6 weeks after stopping chemotherapy);
         - bilateral oophorectomy.

Patients with any of the following were excluded from the trial:
a) patients in whom there was any clinical evidence of metastatic disease;
b) patients who, for whatever reason (e.g. confusion, infirmity, alcoholism), were unlikely to comply with trial requirements;
c) patients whose chemotherapy was started more that 8 weeks (i.e., 56 days) after completion of primary surgery or whose chemotherapy was completed more that 8 weeks (i.e., 56 days) before starting randomised treatment; Chemotherapy if given should be given postoperatively i.e. patients who receive neoadjuvant chemotherapy were ineligible;
d) patients who had not received chemotherapy and whose primary surgery was completed more than 8 weeks (i.e., 56 days) before starting randomised treatment;
e) patients who have received previous hormonal therapy as adjuvant treatment for breast cancer, unless:-
   (i) this was tamoxifen started prior to first surgical procedure and received for less than 29 days; or
   (ii) this was hormonal therapy received pre-surgery in the context of a formal trial, approved by the Steering Committee;
f) patients who have received tamoxifen as part of any breast cancer prevention trials,;
g) patients unwilling to stop taking any drug known to affect sex hormonal status (including HRT), or in whom it would be inappropriate to stop;
h) previous history of invasive breast cancer at any time or other invasive malignancy within the last 10 years, other than squamous or basal cell carcinoma of the skin or carcinoma in situ of the cervix, adequately cone biopsied;
i) any severe concomitant disease which would place the patient at unusual risk or confound the results of the trial, e.g. strong family history of osteoporosis, severe renal or hepatic impairment (defined as AST or ALT greater than three times the upper limit of the reference range); or
j) treatment with non-approved or experimental drug during the 3 months before randomisation;
k) patients considered by the investigator to be at risk of transiting any infection through blood or other body fluids.

The double blind trial used both active and placebo anastrozole and tamoxifen tables, in order to maintain blindness to trial therapy. Anastrozole 1 mg and matching anastrozole placebo were supplied as white, film-coated tablets. Tamoxifen 20mg active tablets and matching tamoxifen placebo were supplied as white, round, biconvex tablets. Patients were randomised to receive one of three oral therapy regimens. The daily dose is one tablet of anastrozole (1mg active or placebo) plus one tablet of tamoxifen (20mg active or placebo). Patients were instructed to take their daily dose at approximately the same time each day. At entry to the trial patients received either a 13-week supply or a 26-week supply of trial material. Patients who received a 13-week supply received a further 13-week supply at the first follow-up visit (3-months). From the 6-month visit onwards, patients received a 26-week supply. In order to assess compliance, patients were asked to return all unused tablets at each visit.

Therapy was started as soon as possible after randomisation, but not more than 8 weeks after completion of surgery and chemotherapy. Patients who have received primary chemotherapy may have started randomised treatment up to a maximum of 8 weeks after completion of chemotherapy or sooner in patients whose blood counts was returning or had returned to normal. Primary chemotherapy must have started within 8 weeks of completing primary surgery. Patients who stop primary chemotherapy before completion of the planned number of cycles may also be randomised. With the exception of the trial therapy, drugs which affect sex hormone status or prevent recurrence of disease were prohibited from use after randomisation until confirmation of disease recurrence. These drugs include the following:-
cytotoxic chemotherapy;
oral adminstration of ketoconazole (antifungal) or related compounds (i.e. topical applications are acceptable);
other hormonal treatments for breast cancer.

A procedure provided that if patients suffer serious menopausal symptoms, e.g., vaginal dryness or bleeding, hot flushes, abdominal cramps, dyspareunia, the following action should be taken:-
a. report the symptom as an adverse event;
b. if patients were willing, carry on with randomised treatment;
c. if necessary, patients may have taken progestins for 3-6 months.

If up to 6 months treatment with progestins failed to control menopausal symptoms, HRT and/or oestrogen creams may be prescribed, and randomised treatment may continue.

The following information was obtained and evaluated from the patient:
a) Date of birth and ethnic origin (recorded at entry);
b) Any relevant past medical history, any concurrent illness, whether or not the patient has had a hysterectomy, previously smoked or had previous HRT (recorded at entry);
c) All concomitant medication/treatment (recorded at entry);
d) Height (measured at entry) and weight (measured at entry and until patient stops randomised treatment);
e) Breast cancer history-Surgical procedure, date of surgery, size and grade of primary tumour, nodal status, ER and PR status (where known), and details of any radiotherapy, chemotherapy and pre-surgical tamoxifen received (recorder at entry);
f) The following routine laboratory assessments was performed at entry: haemoglobin, platelet count, leucocytes, creatinine, total bilirubin, alkakline phosphatase, either asparate aminotransferase or alanine aminotransferase, sodium, potassium and urea. If clinically indicated, further laboratory assessments were made until the patient stops the randomised treatment. Any clinically significant abnormal values should have been recorded as adverse events, unless resulting from disease recurrence. Blood samples were evaluated locally and the laboratory print-outs held in the patient's clinic/hospital records.

Patients were reviewed for recurrence of breast cancer at all follow-up visits, using the following criteria, which are based on British Breast Group recommendations:-

| *SITE OF RECURRENCE* | *METHOD OF CONFIRMATION* |
|---|---|
| LOCO-REGIONAL | |
| 1. Ipsilateral breast (including DCIS) 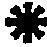 | Histology or cytology |
| 2.Chest wall | Histology or cytology |
| 3. Axillary lymph nodes | Histology or cytology |
| 4. Other regional nodes (i.e. supraclavicular | Histology or cytology |
| and internal mammary) | |

| DISTANT | |
|---|---|
| 5. Skeletal | CT ★ scan, or bone scan with x-ray |
| | evaluation of hot spots. Biopsy may be |
| | necessary in the case of a single lesion. |
| 6. Pulmonary | Chest x-ray |
| 7. Hepatic | CT or US 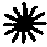 scan |
| 8. Other distant | Imaging and/or biopsy. (Rising tumour |
| | markers alone, eg CA 15.3, are unacceptable) |

| | |
|---|---|
| 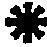 Ductal Carcinoma In Situ ★ Computed Tomography 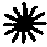 Ultra-sound | |

New breast primaries (either contralateral or ipsilateral) were regarded as disease recurrence events in the statistical analyses of time to recurrence.

The primary statistical endpoint of the trial was time to disease recurrence (locoregional or distant recurrence, new primary breast cancer or death from any cause) and safety/tolerability. The secondary statistical endpoints were time to distant recurrence, time to death, the incidence of new breast primaries and the incidence of pre-defined adverse events. In summary, two treatment comparisons were made: (1) anastrozole alone compared to tamoxifen alone and (2) tamoxifen alone compared to anastrozole and tamoxifen in combination.

For time to disease recurrence, time to distant recurrence and time to death, the primary analysis strategy, included all randomised patients and was performed according to randomised treatment (i.e. 'intention to treat'), with two equally important components:
(i) The log-rank test was used to provide a basic comparison of treatment groups without adjusting for potential prognostic factors. (This is equivalent to fitting the Cox proportional hazards model without adjusting for baseline covariates). 1056 events were required for statistical power.
(ii) The Cox proportional hazards model was fitted, adjusting for baseline receptor status, nodal status, primary tumour size, previous chemotherapy, age and race. A global test of interactions, included all 2 way interactions of each of these prognostic factors with randomised treatment, was undertaken at the 1% level in order to assess whether the treatment comparisons are consistent across different values of the factors. The assumption of proportional hazards was also checked for each prognostic factor using time dependent variables. If there was a departure in the assumption (at the 5% level) then the factor should be included as a stratified variable.

The incidence rates of contralateral breast cancer was formally compared between randomised treatment groups. The primary analysis was based on an intention to treat approach.

A secondary 'per protocol' analysis and a subgroup analysis in patients with oestrogen receptor (ER) positive and/or progesterone receptor (PR) positive tumours, patients with ER negative and PR negative tumours, and all other patients, was also undertaken.

At the major analysis, the incidence rates of the following pre-defined adverse events was formally compared between treatment groups defined by treatment received: hot flushes, nausea and vomiting, asthenia, mood disturbances, musculo-skeletal disorders, vaginal bleeding, vaginal discharge, endometrial cancer, fractures, cataract, venous thromboembolic events, and ischaemic cardiovascular disease. The primary analysis includes all patients who received trial treatment, analysed according to treatment received. A secondary subgroup analysis was also be undertaken, analysed according to trial treatment received, in patients with oestrogen receptor (ER) positive and/or progesterone receptor (PR) positive tumours, patients with ER negative and PR negative tumours, and all other patients remaining safety data was summarised and presented by treatment received. A summary of the methodology is set forth in Figure 3.

Definitions for the statistical analysis:
Time to event (disease recurrence, distant disease recurrence, death) was measured from the date of randomisation. Patients who have not experienced the event at the time of analysis were right-censored at the most recent date of assessment.
Disease recurrence was the earliest of local or distant recurrence new primary breast cancer (contralateral or ipsilateral) or death.
Distant disease recurrence was the earliest of distant recurrence or death.
Disease Stage I at entry was defined as a primary tumour of 2cm or less in its largest dimension and no evidence of lymph node involvement (i.e. the number of positive nodes is zero or axillary surgery was not clinically indicated and therefore was not undertaken). If a patient did satisfy the above criteria, they were considered to be Disease Stage II.

Results of the study are set forth in Figures 1, 2, 3 and 4 and Table 1, 2 and 3. The results relate to a trial consisting of 9,366 patients with a median duration of therapy of 30.7 months with a median follow up of 34.3 months. The total number of first events recorded, i.e. re-emergence of breast cancer, was 1079 of which 766 were in the receptor positive population. The results show that anastrozole is superior to tamoxifen in terms of disease-free survival in the overall population (HR = 0.83) and in the receptor-positive patients (HR = 0.78).

Accordingly, the results of the main analysis of the trial indicate a 17% reduction in the rate of recurrence events (which includes locoregional, distant, and contralateral events, as well as deaths) for the patients receiving anastrozole vs. those receiving tamoxifen. Within the hormone receptor positive (oestrogen receptor positive and/or progesterone receptor positive) patients, there was a 22% reduction in recurrence rate for anastrozole vs. tamoxifen patients.

The results also show that anastrozole is superior to tamoxifen in terms of the incidence of contralateral breast cancer in the overall population (OR = 0.42). For the incidence of new (contralateral) breast primary tumours the odds were 0.42 for anastrozole when compared to tamoxifen (a value of 1.00 would show equivalence) in the overall population.

Anastrozole was significantly better tolerated (vs. tamoxifen) with respect to endometrial cancer, vaginal bleeding, vaginal discharge, ischemic cerebrovascular events, venous thromboenbolic events, hot flushes and weight gain. Tamoxifen was better tolerated with respect to musculoskeletal disorders and fractures.

**Table 1- Patient Characteristics**

| | **Anastrozole** | **Tamoxifen** | **Combination** |
|---|---|---|---|
| | **(n=3125)** | **(n=3116)** | **(n=3125)** |
| **Mean age (years)** | 64.1 | 64.1 | 64.3 |
| **Mean weight (kg)** | 70.8 | 71.1 | 71.3 |

| **Receptor status (%)** | | | |
|---|---|---|---|
| **Positive** | 83.7 | 83.3 | 84.0 |
| **Negative** | 7.4 | 8.0 | 6.9 |
| **Other** | 8.9 | 8.7 | 9.1 |

| **Primary treatment (%)** | | | |
|---|---|---|---|
| **Mastectomy** | 47.8 | 47.3 | 48.1 |
| **Axillary surgery** | 95.5 | 95.7 | 95.2 |
| **Radiotherapy** | 63.3 | 62.5 | 62.0 |
| **Chemotherapy** | 22.3 | 20.8 | 20.8 |
| **Prior tamoxifen** | 1.6 | 1.7 | 1.7 |

**Table 2- Disease Characteristics**

| | **Anastrozole** | **Tamoxifen** | **Combination** |
|---|---|---|---|
| | **(n=3125)** | **(n=3116)** | **(n=3125)** |
| **Primary tumour size (%)** | | | |
| **T1 (≤2cm)** | 63.9 | 62.9 | 64.1 |
| **T2 (>2cm and ≤5cm)** | 32.6 | 34.2 | 32.9 |
| **T3 (>5cm)** | 2.7 | 2.2 | 2.3 |

| **Nodal status (%)** | | | |
|---|---|---|---|
| **Node +ve** | 34.9 | 33.6 | 33.5 |

| **Grading (%)** | | | |
|---|---|---|---|
| **Well differentiated** | 20.8 | 20.5 | 21.2 |
| **Moderately differentiated** | 46.8 | 47.8 | 46.6 |
| **Poorly/undifferentiated** | 23.7 | 23.3 | 23.7 |
| **Not assessed/recorded** | 8.7 | 8.4 | 8.5 |

**Table 3 - First events in ITT population**

| | **Anastrozole** | **Tamoxifen** | **Combination** |
|---|---|---|---|
| | **(n=3125)** | **(n=3116)** | **(n=3125)** |
| **First event** | 317 | 379 | 383 |
| **Locoregional** | 67 | 83 | 81 |
| **Distant** | 156 | 181 | 202 |
| **Contralateral (invasive)** | 9 | 30 | 23 |
| **Contralateral (DCIS)** | 5 | 3 | 5 |
| **Death - breast cancer** | 2 | 1 | 2 |
| **Death - other reason** | 78 | 81 | 70 |

## Claims

1. The use of anastrozole, or a pharmaceutically acceptable salt thereof, in the preparation of a medicament for the reduction of the rate of recurrence of cancer in a post-menopausal woman having early breast cancer.

2. The use of anastrozole, or a pharmaceutically acceptable salt thereof, in the preparation of a medicament for the reduction of the rate of a new contralateral primary tumour in a post-menopausal woman having early breast cancer.

3. The use according to Claim 1 or Claim 2 wherein the anastrozole is provided in the absence of an anti-oestrogen agent.

4. The use according to Claim 3 wherein the anti-oestrogen agent is tamoxifen .

5. The use according to any one of the preceding claims wherein the woman having said early breast cancer is oestrogen receptor positive or progesterone receptor positive.

6. The use according to Claim 5 wherein the woman having said early breast cancer is oestrogen receptor positive and progesterone receptor positive.

7. The use according to any one of the preceding claims wherein the anastrozole is provided for administration at a dose of 1mg/per day.

## Patentansprüche

1. Verwendung von Anastrozol oder eines pharmazeutisch annehmbaren Salzes davon bei der Herstellung eines Medikaments zur Reduzierung der Rezidivrate von Krebs bei einer postmenopausalen Frau mit Brustkrebs im Frühstadium.

2. Verwendung von Anastrozol oder eines pharmazeutisch annehmbaren Salzes davon bei der Herstellung eines Medikaments zur Reduzierung der Rate eines neuen kontralateralen Primärtumors bei einer postmenopausalen Frau mit Brustkrebs im Frühstadium.

3. Verwendung nach Anspruch 1 oder Anspruch 2, worin Anastrozol in Abwesenheit eines Anti-Östrogens bereitgestellt wird.

4. Verwendung nach Anspruch 3, worin das Anti-Östrogen Tamoxifen ist.

5. Verwendung nach einem der vorhergehenden Ansprüche, worin die Frau mit Brustkrebs im Frühstadium östrogenrezeptor-positiv oder progesteronrezeptornegativ ist.

6. Verwendung nach Anspruch 5, worin die Frau mit Brustkrebs im Frühstadium östrogenrezeptor-positiv und progesteronrezeptor-positiv ist.

7. Verwendung nach einem der vorhergehenden Ansprüche, worin Anastrozol in einer Dosis von 1 mg/Tag verabreicht wird.

## Revendications

1. Utilisation d'anastrozole, ou d'un sel pharmaceutiquement acceptable de celui-ci, pour la préparation d'un médicament destiné à réduire le taux de récurrence d'un cancer chez une femme postménopausée atteinte d'un cancer du sein à un stade précoce.

2. Utilisation d'anastrozole, ou d'un sel pharmaceutiquement acceptable de celui-ci, pour la préparation d'un médicament destiné à réduire le taux de nouvelle tumeur primaire controlatérale chez une femme postménopausée atteinte d'un cancer du sein à un stade précoce.

3. Utilisation selon la revendication 1 ou la revendication 2, selon laquelle l'anastrozole est fourni en l'absence d'agent anti-oestrogène.

4. Utilisation selon la revendication 3, selon laquelle l'agent anti-oestrogène est le tamoxifène.

5. Utilisation selon l'une quelconque des revendications précédentes, selon laquelle la femme atteinte dudit cancer du sein à un stade précoce est positive pour les récepteurs aux oestrogènes ou positive pour les récepteurs à la progestérone.

6. Utilisation selon la revendication 5, selon laquelle la femme atteinte dudit cancer du sein à un stade précoce est positive pour les récepteurs aux oestrogènes et positive pour les récepteurs à la progestérone.

7. Utilisation selon l'une quelconque des revendications précédentes, selon laquelle l'anastrozole est administré à une dose de 1 mg/jour.
